Europäisches Patentamt

European Patent Office

Office européen des brevets

(19)

(11) Publication number : **0 136 949 B2**

# NEW EUROPEAN PATENT SPECIFICATION

(12)

(45) Date of publication of the new patent specification : **08.07.92 Bulletin 92/28**

(51) Int. Cl.⁵ : **A61B 17/064,** A61B 17/072

(21) Application number : **84401937.2**

(22) Date of filing : **27.09.84**

(54) **Surgical fastener retainer member assembly.**

(30) Priority : **04.10.83 US 538988**

(43) Date of publication of application :
**10.04.85 Bulletin 85/15**

(45) Publication of the grant of the patent :
**24.06.87 Bulletin 87/26**

(45) Mention of the opposition decision :
**08.07.92 Bulletin 92/28**

(84) Designated Contracting States :
**AT BE CH FR IT LI NL SE**

(56) References cited :
**EP-A- 0 120 794
EP-A- 0 130 037
WO-A-83/01193
FR-A- 322 578
FR-A- 2 452 275**

(56) References cited :
**US-A- 198 982
US-A- 3 166 072
US-A- 3 357 296
US-A- 3 924 629
US-A- 4 060 089
US-A- 4 304 743
US-A- 4 379 457**

(73) Proprietor : **United States Surgical Corporation
150 Glover Avenue
Norwalk, Connecticut 06856 (US)**

(72) Inventor : **Green, David T.
251 Wolfpit Avenue
Norwalk Connecticut 06851 (US)**

(74) Representative : **Marsh, Roy David et al
Hoffmann Eitle & Partner Patent- und
Rechtsanwälte Arabellastrasse 4 Postfach 81
04 20
W-8000 München 81 (DE)**

EP 0 136 949 B2

Jouve, 18, rue Saint-Denis, 75001 PARIS

## Description

### Background of the Invention

The invention relates to surgical fasteners, and more particularly to a retainer member assembly for facilitating application of such surgical fasteners to body tissue.

Surgical stapling devices allow a surgeon to fasten body tissue by applying surgical staples. The staples may be applied singly in succession or a number may be applied simultaneously. Surgical staples are often made of metals such as tantalum or stainless steel, which are inert. Fasteners of magnesium, which fasteners are gradually absorbed by the body, are also known.

Non-metallic fasteners are also known and in some cases may have certain advantages over metal staples. For example, metal staples in the body may scatter X-rays and may therefore degrade the quality of radiographs.

However, metal staples also have certain advantages over non-metallic fasteners. They can be bent or crimpled and will hold their new shape in or around tissue. In contrast, objects of non-metallic resinous materials are usually too resilient (i.e., elastic) to hold deformed shapes (assuming plastic flow does not occur). (As used herein, the terms "resinous materials" means non-metallic materials, such as natural or synthetic polymers and resins, including protein-based materials, which are relatively flexible and elastic, and which may or may not be absorbable in the body).

To circumvent this characteristic of resinous materials, surgical fasteners of these materials may be made in two parts: a fastener member and a retainer member. The prong or prongs of the fastener member are driven through one side of the tissue to be fastened and the retainer member interlocks with the prongs of the fastener member on the other side of the tissue to hold the entire fastener structure in place. One such fastener structure and apparatus for applying it are disclosed in Green U.S. patent 4,402,445, issued September 6, 1983, which is hereby incorporated by reference in its entirety.

Such fastener structures are relatively small in size and typically are applied to tissue several at a time in rows. Because of their small size, loading the fastener and retainer members into the fastener-apparatus can be both tedious and time-consuming. The need exists for fastener structures that can be loaded quickly and easily.

US-A-3 357 296 discloses a unitary array of pronged staples and a unitary array of corresponding staple receiver elements, the arrays being for loading in to a stapler which is in overall operation similar to conventional staplers with a knife severing a web connecting the leading staple to the array of staples, and the leading receiver to the array of receivers, on each actuation of the stapler.

US-A-4 060 089 discloses a multi-pronged surgical fastener strip and a retainer strip having a corresponding multiplicity of apertures to receive respective prongs of the fastener strip. The strips may be bio-absorbable and the prongs are barbed.

US-A-3 166 072 discloses a strip of tape to which is attached a line of discrete clips each of which functions both as a spiked fastener and a slotted retainer, its slot retaining the spike of clip in opposition to it. After use in surgery of two such strips of clips, the clips are present as pairs, the members of each pair being coupled through clipped tissue between them, and the two backing tape strips are removed.

### Summary of the Invention

In accordance with this invention, a surgical fastener in accordance with claim 1 is provided. The assembly comprises (1) a plurality of surgical fastener retainer members arranged in a configuration which has surgical utility (e.g., two parallel rows of members) and (2) yieldable (i.e. flexible or frangible) links, preferably of the same material as the retainer members, disposed between adjacent retainer members so that each retainer member is connected by at least one such link to at least one other retainer member in the array, all the retainer members thereby being connected directly or indirectly to each other. The present invention finds its greatest use with surgical fasteners of resinous materials, although the invention may be used with fasteners of other materials that have separate retainer members.

This assembly facilitates handling surgical fastener retainer members and greatly reduces the time and tedium involved in loading retainer members into fastener-applying apparatus; only a single loading of all the retainers at one time is required.

### Brief Description of the Drawings

The above and other advantages of the present will be more apparent after consideration of the accompanying drawings in which like parts are indicated by like reference characters throughout and in which:

FIG. 1 is an exploded perspective view of an individual surgical fastener of the type with which the present invention is used;

FIG. 2 is a fragmentary exploded perspective view showing a typical latch means for retaining individual surgical fastener retainer members on a fastener-applying apparatus;

FIG. 3 is a fragmentary plan view of individual surgical fastener retainer members retained on the applying apparatus only by the latch means of FIG. 2;

FIG. 4 is a fragmentary plan view of a surgical fastener retainer member assembly according to the present invention, which assembly is being retained in the applying apparatus;

FIG. 5 is a plan view of the surgical fastener retainer member assembly of FIG. 4; and

FIGS 6A-6E are schematic plan views of five alternate configurations of surgical fastener retainer member assemblies according to the present invention.

Detailed Description of the Invention

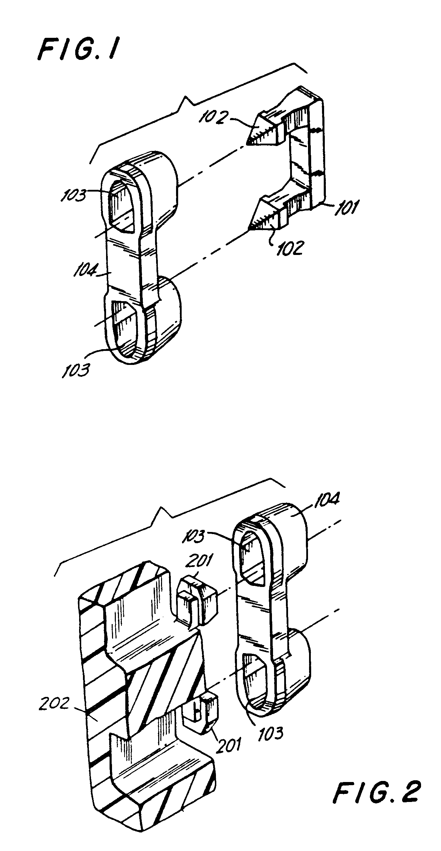

A surgical fastener of the type with which the present invention can be used is shown in FIG. 1. The fastener includes fastener member 101, which has two prongs 102 that are driven through tissue to engage apertures 103 in retainer member 104. Both members may be made from a resinous material which may or may not be absorbable in the body.

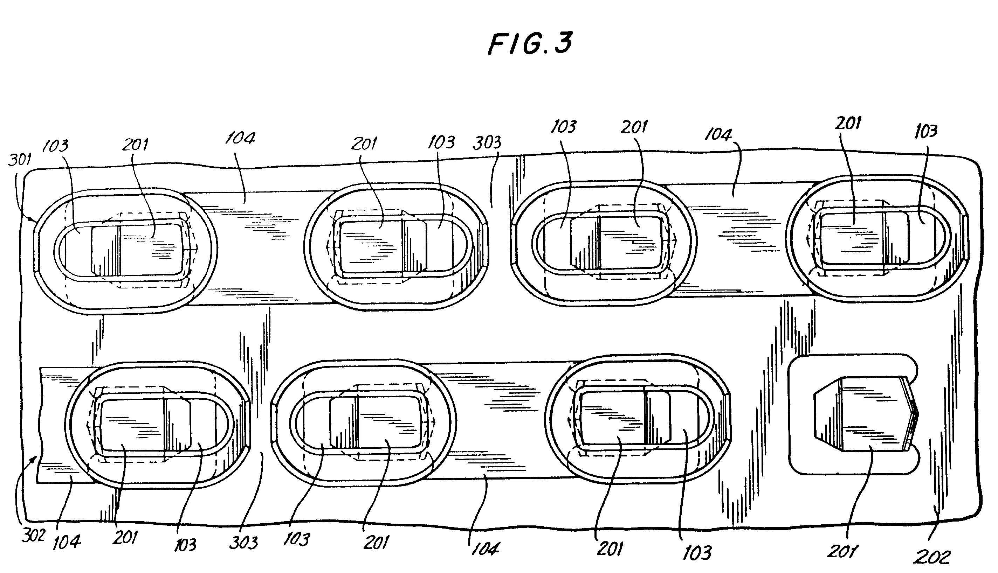

These fasteners are applied either simultaneously or in succession in a surgically useful configuration to join tissue. One such configuration is shown in FIG. 3. This configuration is useful because it is linear and it provides good hemostasis. The latter is provided by the offset between rows 301 and 302 whereby the fasteners in one row clamp most tightly the tissue opposite gaps 303 in the other row.

The fasteners are applied using apparatus specifically designed for that purpose. As shown in FIGS. 2 and 3 for one such apparatus, lugs 201 engage apertures 103 to hold retainer member 104 securely to portion 202 of the body of the apparatus. Entry of prongs 102 into apertures 103 disengages lugs 201 at the moment at which lugs 201 are no longer needed to secure retainer members 104 to the apparatus. As explained above, each retainer member 104 must be individually loaded on lugs 201, in a tedious and time-consuming procedure.

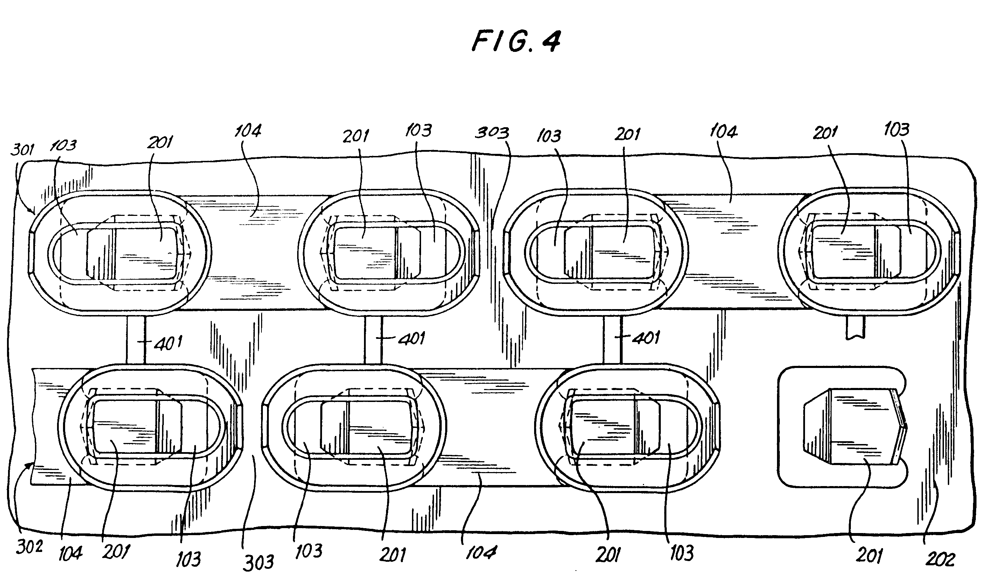

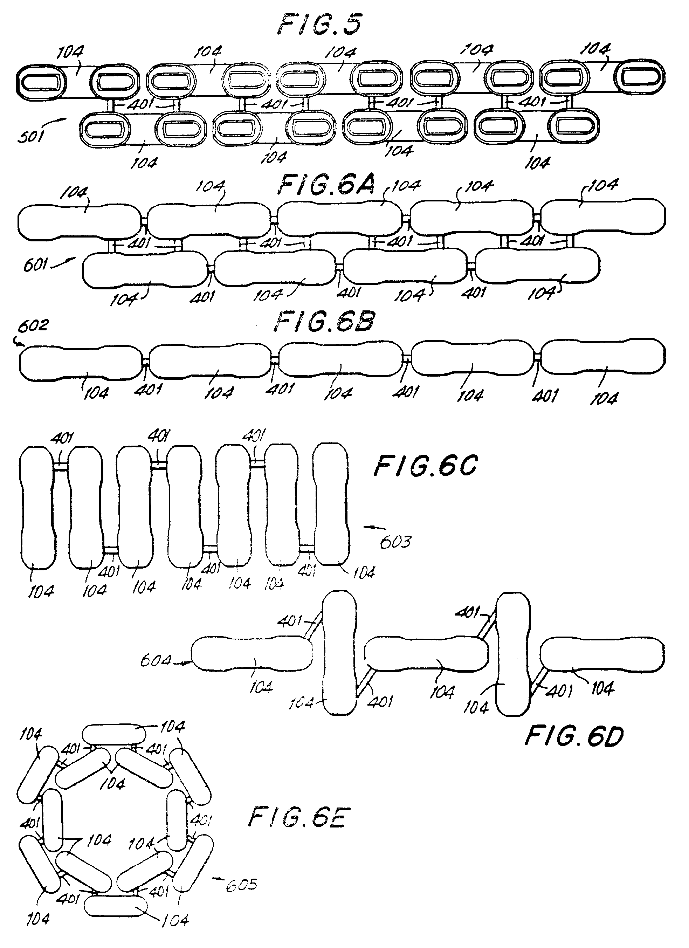

This procedure is expedited by the provision of a unitary assembly of retainer members. The retainer members are connected as shown in FIG. 4 by yieldable links 401, formed preferably of the same material as the retainer members. Retainer member assembly 501 of seven retainer members 104 is shown in FIG. 5. The number of retainer members in an assembly and the assembly configuration will vary depending on the particular surgical application. Other possible configurations 601, 602, 603, 604, and 605 are shown schematically in FIGS. 6A-6E. In particular, in configuration 601 of FIG. 6A, each retainer member is connected to adjacent retainer members by two links at each end thereof.

Yieldable links 401 must be capable of maintaining the integrity of assembly 501 during handling and loading but should be sufficiently flexible or frangible (i.e., breakable) so that once the fasteners are inserted into tissue, the links will yield sufficiently to flex with the fastened tissue. Specifically, when the surgical fasteners are applied the configuration of the fastener assembly will usually be planar. However, afterwards the fastened tissue may become non-planar. Links 401 should be sufficiently yieldable to allow the fastener array to follow the movement of the tissue. Accordingly, the links must either break or be flexible enough to allow relative motion among the fasteners.

Other benefits of the links are numerous. If a retainer member 104 is not properly latched into the applying apparatus 202, it still will be held in place by the neighbouring retainer members 104 through the links 401. Similarly, if the surgeon inadvertently actuates the apparatus when not all of the fastener-/retainer pairs are positioned to engage tissue, the mated fastener/retainer pairs that do not engage tissue will be connected to those mated fastener/retainer pairs that do engage tissue. The former remain associated with the fastened tissue and are therefore readily visible to the surgeon who can easily remove them by breaking the links with an instrument or by hand. Finally, the larger size of the assembly as compared to the size of the individual members makes the handling of the retainer members easier during the loading operation.

In summary, the surgical fastener retainer member assembly of this invention facilitates handling of surgical fastener retainer members and their loading into surgical fastener-applying instruments. The assembly of this invention provides for securing the retainer members in the instruments. One skilled in the art will recognize that the inventive principles disclosed herein can be practiced in other than the embodiments described, and the invention is not limited by those embodiments but only by the claims which follow.

## Claims

1. A surgical fastener comprising a plurality of pronged fastener members and a unitary assembly of surgical fastener retainer members, said assembly comprising :

said plurality of surgical fastener retainer members (104) arranged in a surgically useful configuration and each having two spaced apart apertures to receive the prongs of the pronged fastener members, to form a fastener array; and

one or more links (401), disposed between the retainer members (104), each of said retainer members being connected by at least one such link to at least one other retained member so that all the retainer members are connected to each other to form said unitary assembly;

the fastener being characterised in that:

the links are yieldable, that is, they must either break or be flexible enough to allow relative motion among the fastener members, to allow the fastener array to follow the movement of fastened tissue.

2. The fastener of claim 1 wherein the yieldable link (401) is frangible.

3. The fastener of claim 1 wherein the configuration (602) is linear.

4. The fastener of claim 3 wherein the linear configuration comprises a first row (301) of retainer members (104) and a second row (302) of retainer members (104) parallel and adjacent to the first row (301), the second row (302) offset linearly with respect to the first row (301).

5. The fastener of claim 1 wherein the configuration (605) is circular comprises and comprises a first circle of retainer members (104) and a second circle of retainer members (104) concentric with the first circle, the second circle offset angularly with respect to the first circle.

6. The fastener of claim 1 wherein the retainer members (104) are made of a resinous material.

7. The fastener of claim 7 wherein the resinous material is absorbable in the body.

8. The fastener of claim 1 wherein the link (410) is made of the same material as the retainer members (104).

9. In combination:

apparatus for applying two-part surgical fasteners positioned within the apparatus each of the fasteners having a plumbits of pronged fastener members (101) and

a corresponding number of retainer members (104) in a unitary assembly arranged in a surgically useful configuration, each retainer member having two spaced-apart to receive the pronges of the pronged fastener members, to form a fastener array; and

one or more links (401) disposed between the retainer members (104), each of the retainer members being connected by at least one such link to at least one other retainer member so that all the retainer members are connected to each other to form said unitary assembly;

the fastener being characterised in that:

the links are yieldable, that is, they must either break or be flexible enough to allow relative movement among the fastener members, to allow the fastener array to follow the movement of fastened tissue.

## Patentansprüche

1. Chirurgische Befestigungseinrichtung, umfassend eine Vielzahl von gezinkten Befestigungselementen und eine unitäre Anordnung von chirurgischen Befestigungs-Aufnahmeelementen, wobei die Anordnung umfaßt:

die Vielzahl von chirurgischen Befestigungs-Aufnahmeelementen (104), die in einer chirurgisch brauchbaren Konfiguration angeordnet sind und wobei jedes zwei voneinander beabstandete Öffnungen aufweist, um die Zinken des gezinkten Befestigungselementes aufzunehmen, um ein Befestigungseinrichtungsfeld zu bilden; und

eine oder mehrere Verbindungen (401), die zwischen den Aufnahmeelementen (104) angeordnet sind, wobei jedes dieser Aufnahmeelemente durch wenigstens eine derartige Verbindung mit wenigstens einem anderen Aufnahmeelement verbunden ist, so daß alle Aufnahmeelemente miteinander verbunden sind, um die unitäre Anordnung zu schaffen;

dadurch gekennzeichnet, daß

die Verbindungen nachgebend sind, das heißt, sie müssen entweder brechen oder ausreichend biegsam sein, um eine relative Bewegung unter den Befestigungselementen zu erlauben, um zu erlauben, daß die Befestigungsanordnung der Bewegung des befestigten Gewebes folgt.

2. Befestigungseinrichtung nach Anspruch 1, dadurch gekennzeichnet, daß die nachgebende Verbindung (401) brechbar ist.

3. Befestigungseinrichtung nach Anspruch 1, dadurch gekennzeichnet, daß die Konfiguration (602) linear ist.

4. Befestigungseinrichtung nach Anspruch 3, dadurch gekennzeichnet, daß die lineare Konfiguration eine erste Reihe (301) von Aufnahmeelementen (104) und eine zweite Reihe (302) von Aufnahmeelementen (104) parallel und benachbart zu der ersten Reihe (301) umfaßt, wobei die zweite Reihe (302) bezüglich der ersten Reihe (301) linear verschoben ist.

5. Befestigungseinrichtung nach Anspruch 1, dadurch gekennzeichnet, daß die Konfiguration (605) kreisförmig ist und einen ersten Kreis von Aufnahmeelementen (104) und einen zweiten Kreis von Aufnahmeelementen (104) konzentrisch mit dem ersten Kreis umfaßt, wobei der zweite Kreis bezüglich des ersten Kreises winkelmäßig verschoben sind.

6. Befestigungseinrichtung nach Anspruch 1, dadurch gekennzeichnet, daß die Aufnahmeelemente (104) aus einem kunstharzartigen Material hergestellt sind.

7. Befestigungseinrichtung nach Anspruch 7, dadurch gekennzeichnet, daß das kunstharzartige Material im Körper absorbierbar ist.

8. Befestigungseinrichtung nach Anspruch 1, dadurch gekennzeichnet, daß die Verbindung (410) aus demselben Material hergestellt ist wie die Aufnahmeelemente (104).

9. In Kombination:

Vorrichtung zur Anbringung von zweiteiligen chirurgischen Befestigungseinrichtungen, die innerhalb der Vorrichtung positioniert sind, wobei jede der Befestigungseinrichtungen eine Vielzahl von gezinkten Befestigungselementen (101) und eine entsprechende Anzahl von Aufnahmeelementen (104) in einer unitären Anordnung besitzt, die in einer chirurgisch brauchbaren Konfiguration angeordnet sind, wobei jedes Aufnahmeelement zwei voneinander beabstandete Öffnungen aufweist, um die Zinken der gezinkten Befestigungselemente aufzunehmen, um ein Befestigungseinrichtungsfeld zu bilden; und

eine oder mehrere Verbindungen (401), die sich zwischen den Aufnahmeelementen (104) befinden, wobei jedes der Aufnahmeelemente durch wenigstens eine derartige Verbindung mit wenigstens einem anderen Aufnahmeelement so verbunden sind, daß alle Aufnahmeelemente miteinander verbunden sind, um die unitäre Anordnung zu bilden;

dadurch gekennzeichnet, daß

die Verbindungen nachgebend sind, das heißt sie müssen entweder brechen oder biegsam genug sein, um eine relative Bewegung unter den Befestigungselementen zu erlauben, um zu erlauben, daß die Befestigungsanordnung der Bewegung von befestigtem Gewebe folgt.

## Revendications

1. Agrafe chirurgicale comprenant une pluralité d'éléments à dents et un ensemble unitaire d'éléments de retenue d'agrafes chirurgicales, ledit ensemble comprenant :

ladite pluralité d'éléments de retenue d'agrafes chirurgicales (104) agencée en une configuration chirurgicalement utile, chacun ayant deux ouvertures espacées pour recevoir les dents des éléments d'agrafe à dents, pour former une configuration d'agrafes ; et

une ou plusieurs barrettes (401) disposées entre les éléments de retenue (104), chacun desdits éléments de retenue étant connecté par au moins une telle barrette à au moins un autre élément de retenue du manière que tous les éléments de retenue soient connectés les uns aux autres pour former ledit ensemble unitaire ;

l'agrafe étant caractérisée en ce que

les barrettes peuvent céder c'est-à-dire dire qu'uelles doivent soit se casser ou bien être suffisamment flexibles pour permettre un mouvement relatif parmi les éléments d'agrafes afin de permettre à la configuration des agrafes de suivre le mouvement du tissu agrafé

2. Agrafe selon la revendication 1 où la barrette qui peut céder (401) est cassable.

3. Agrafe selon la revendication 1 où la configuration (602) est linéaire.

4. Agrafe de la revendication 3 où la configuration linéaire comprend un première rangée (301) d'éléments de retenue (104) et une seconde rangée (302) d'éléments de retenue (104) parallèle et adjacente à la première rangée (301), la seconde rangée (302) étant linéairement décalée par rapport à la première rangée (301).

5. Agrafe de la revendication 1 où la configuration (605) est circulaire et comprend un premier cercle d'éléments de retenue (104) et un second cercle d'éléments de retenue (104) concentrique avec le premier cercle, le second cercle étant angulairement décalé par rapport au premier cercle.

6. Agrafe de la revendication 1 où les éléments de retenue (104) sont faits en un matériau à base de résine.

7. Agrafe de la revendication 1 où le matériau à base de résine est absorbable par le corps.

8. Agrafe de la revendication 1 où la barrette (401) est faite du même matériau que les éléments de retenue (104).

9. En combintion :

appareil pour appliquer des agrafes chirurgicales en deux pièces placées dans l'appareil, chacune des agrafes ayant une pluralité d'éléments d'agrafe à dents (101); et

un nombre correspondant d'éléments de retenue (104) en un ensemble unitaire agencé en une configuration chirurgicalement utile, chaque élément de retenue ayant deux ouvertures espacées pour recevoir les dents des éléments de retenue à dents afin de former une configuration d'agrafes,

une ou plusieurs barrettes (401) disposées entre les éléments de retenue (104), chacun des éléments de retenue étant connecté par au moins une telle barrette à au moins un autre élément de retenue de manière que tous les éléments de retenue soient connectés l'un à l'autre pour former un ensemble unitaire, ladite agrafe étant caractérisée en ce que les barrettes peuvent céder, c'est-à-dire qu'elles doivent soit se casser ou bien être suffisamment flexibles pour permetttre un mouvement relatif parmi les éléments d'agrafage afin de permettre à la configuration d'agrafes de suivre le mouvement du tissu agrafé.

FIG. 1

FIG. 2

# FIG.3

FIG.4

EP 0 136 949 B2

FIG.5

FIG.6A

FIG.6B

FIG.6C

FIG.6D

FIG.6E